# EUROPÄISCHE PATENTSCHRIFT

(11) **EP 1 139 995 B1**
(45) Veröffentlichungstag und Bekanntmachung des Hinweises auf die Patenterteilung: **24.11.2004**
(21) Anmeldenummer: 99966934.4
(22) Anmeldetag: 09.12.1999
(51) Int. Cl.: A61K 7/16, A61K 7/18

(54) **FEINTEILIGE SUSPENSIONEN SCHWERLÖSLICHER CALCIUMSALZE UND DEREN VERWENDUNG IN ZAHNPFLEGEMITTELN**
FINE SUSPENSIONS OF POORLY SOLUBLE CALCIUM SALTS AND THEIR USE IN DENTAL CARE PRODUCTS
SUSPENSIONS DE SELS DE CALCIUM DE FINE DISPERSION ET PEU SOLUBLES DANS L'EAU ET LEUR UTILISATION DANS DES PRODUITS D'HYGIENE DENTAIRE

(30) Priorität: 18.12.1998 DE 19858662
(43) Veröffentlichungstag der Anmeldung: 10.10.2001
(73) Patentinhaber: Henkel Kommanditgesellschaft auf Aktien, 40589 Düsseldorf-Holthausen (DE)
(72) Erfinder: KROPF, Christian, D-40597 Düsseldorf (DE); BRÜNINGHAUS, Ulrike, D-40789 Monheim (DE); PASTURA, Amerigo, D-58453 Witten (DE); HEMPELMANN, Rolf, D-66386 St. Ingbert (DE); MEINDERS, Michael, D-47800 Krefeld (DE); ROTH, Marcel, D-40597 Düsseldorf (DE); WÜLKNITZ, Peter, D-42799 Leichlingen (DE)
(86) Internationale Anmeldenummer: PCT/EP1999/009683
(87) Internationale Veröffentlichungsnummer: WO 2000/037033

(56) Entgegenhaltungen:
- EP-A- 0 208 790
- EP-A- 0 732 343
- GB-A- 1 110 900
- GB-A- 2 206 338

## Beschreibung

Die Erfindung betrifft feinteilige Suspensionen schwerlöslicher Calciumsalze, die aufgrund ihrer Teilchengröße im Nanometerbereich und ihrer Stabilität gegen Agglomeration sich besonders für die Anwendung in Zahnpflegemitteln eignen.

Phosphatsalze des Calciums werden seit langem sowohl als Abrasivkomponenten als auch zur Förderung der Remineralisierung des Zahnschmelzes den Rezepturen von Zahnreinigungsmitteln und Zahnpflegemitteln zugesetzt. Dies gilt insbesondere für Hydroxylapatit und Fluorapatit sowie für amorphe Calciumphosphate und für Brushit (Dicalciumphosphat-dihydrat). Aber auch Calciumfluorid ist als Bestandteil von Zahnreinigungsmitteln und als Komponente zur Festigung des Zahnschmelzes und zur Kariesprophylaxe mehrfach beschrieben worden.

Die Verfügbarkeit dieser Substanzen für die erwünschte Remineralisierung hängt ganz entscheidend von der Teilchengröße dieser in Wasser schwerlöslichen und in den Zahnpflegemitteln dispergierten Komponenten ab. Man hat daher vorgeschlagen, diese schwerlöslichen Calciumsalze in feinster Verteilung einzusetzen.

Aus DE-A-2134862 war z.B. ein Zahnpflegemittel für hypersensible Zähne bekannt, das feinstteiligen Hydroxylapatit (Ca₅[(PO₄)₃OH]) enthält, dessen Teilchengröße aber mit 6 - 8 µm (Mikrometer) angegeben wird, da durch Mahlung kaum höhere Feinheiten erzielbar sind.

Man hat auch bereits Zahnpflegemittel aus getrennten Komponenten, von denen die eine ein gelöstes Ca-Salz und die andere ein gelöstes Phosphat- oder Fluoridsalz enthält, und die erst kurz vor ihrer Anwendung vereinigt werden - oder die nacheinander angewendet werden - vorgeschlagen, um die frisch gefällten und noch amorphen oder feinkristallinen Calciumsalze an die Zahnoberfläche zu bringen. Die Nachteile einer solchen Handhabung liegen auf der Hand, da der Verwender zwei Produkte nacheinander anwenden oder diese kurz vor der Verwendung vereinigen muß. Werden Zusammensetzungen, die frisch gefällte, noch amorphe Calciumphosphate oder Calciumfluorid enthalten, gelagert, so altern die Niederschläge, die Kristallite wachsen und agglomerieren zu gröberen Sekundärpartikeln. Dadurch wird die remineralisierende Wirkung verringert und die Stabilität der Dispersion gefährdet.

Es bestand daher die Aufgabe, Suspensionen solcher schwerlöslicher Calciumsalze bereitzustellen, deren Partikelgröße im Nanometerbereich liegt und die gegen Agglomeration weitgehend geschützt sind.

In WO 94/04460 A1 ist ein Verfahren zur Herstellung amorpher Calciumsalze und deren Verwendung zur Remineralisierung der Zähne beschrieben. In EP 786245 A1 werden Zahnpflegemittel beschrieben, die Hydroxylapatit mit Teilchengrößen von 0,05 bis 1,0 µm, die durch Mahlen erhalten werden, enthalten. Aus WO 98/18719 ist eine Hydroxylapatit-Zusammensetzung bekannt, die Hydroxylapatit mit Teilchendurchmessern von 10 - 20 nm und Teilchenlängen von 50 - 100 nm enthalten und die z.B. in Zahnpasten verwendet werden sollen. Diese werden durch Aufkonzentrierung von sehr verdünnten Suspensionen durch mehrere Filtrationsschritte erhalten.

Aus EP 0499299 A2 sind Suspensionen von Partikeln kristalliner Drogen bekannt, die eine Größe von weniger als 100 nm aufweisen und auf ihrer Oberfläche einen Oberflächenmodifikator adsorbiert enthalten, der auch ein Tensid oder ein polymeres Schutzkolloid sein kann. Eine Stabilisierung anorganischer, durch Fällungsreaktionen erhaltener, schwerlöslicher Salze wird nicht offenbart. Aus WO 96/34829 A1 ist ein Verfahren zur Herstellung wenig agglomerierter Teilchen im Nanometerbereich bekannt, bei welchem eine Suspension solcher Teilchen aus den Precursoren in einem flüssigen Medium, das für die Partikel kein nennenswertes Lösevermögen aufweist, in Gegenwart einer oberflächenblockierenden Substanz hergestellt wird. In einer anderen Ausführungsform wird ein Sol, das amorphe oder teilkristalline Nanopartikel enthält, in Gegenwart der oberflächenblockierenden Substanz suspendiert. Als oberflächenblockierende Substanzen werden auch (Poly)carbonsäuren und nichtionogene Tenside genannt. Als geeignete Teilchen werden jedoch nur Oxid(hydrate), Sulfide, Selenide, Telluride und Phosphide offenbart, die aus hydrolysierbaren Salzen oder metallorganischen Verbindungen durch Wasserzusatz oder pH-Änderung ausgefällt werden. Phosphate oder Fluoride des Calciums oder eine Verwendung der Suspensionen in Zahnpflegemitteln sind nicht offenbart.

Es wurde nun gefunden, daß sich auch Suspensionen von in Wasser schwerlöslichen Calciumsalzen in sehr feinteiliger Form während der Ausfällung oder kurz danach stabilisieren lassen, wenn man die Ausfällung in Gegenwart eines Agglomerations-Inhibitors durchführt oder die Dispersion in Gegenwart des Agglomerationsinhibitors redispergiert.

Gegenstand der Erfindung ist daher eine Suspension von wenig wasserlöslichen Calciumsalzen, ausgewählt aus Phosphaten, Fluoriden und Fluorphosphaten in einem flüssigen Medium, in dem diese Calciumsalze nicht oder wenig löslich sind, dadurch gekennzeichnet, daß die Calciumsalze in Form von Primärteilchen mit Durchmessern von 5 bis 50 Nanometern (nm) und Längen von 10 bis 150 Nanometern vorliegen und durch einen Gehalt von wenigstens 0,01 Gew.-%, bezogen auf das Gewicht der Suspension, eines wasserlöslichen Tensids oder eines wasserlöslichen polymeren Schutzkolloids gegen Agglomeration stabilisiert sind.

Als wenig löslich bzw. wenig wasserlöslich sollen solche Salze verstanden werden, die in Wasser bzw. in dem flüssigen Suspensionsmedium zu weniger als 1 g/l (20° C) löslich sind. Bevorzugt geeignete Salze sind Calciumhydroxyphosphat (Ca₅[OH(PO₄)₃]) bzw. Hydroxylapatit, Calciumfluorphosphat (Ca₅[F(PO₄)₃]) bzw. Fluorapatit, F-dotierter Hydroxylapatit der allgemeinen Zusammensetzung Ca₅(PO₄)₃(OH,F) und Calciumfluorid (Ca F₂) bzw. Fluorit (Flußspat).

Als flüssiges Medium, in welchem die Calciumsalze dispergiert sein können, ist in erster Linie Wasser geeignet. Die aus einer wäßrigen Suspension z.B. durch Filtration oder Zentrifugation isolierten Calciumsalz-Partikel können aber auch in organischen Solventien redispergiert werden und liefern dabei ebenfalls Suspensionen der Primärpartikel im Nanometerbereich, die kaum zur Agglomeration neigen. Geeignete organische flüssige Medien sind z.B. wasserlösliche, niedere Alkohole und Glycole, Polyethylenglycole, Glycerin oder deren Mischungen untereinander oder mit Wasser.

Unter Primärteilchen werden hier die Kristallite, d.h. die Einzelkristalle der genannten Calciumsalze verstanden. Als Teilchendurchmesser soll hier der kleinste Durchmesser, als Länge der größte Durchmesser der Kristallpartikel z.B. die Länge eines stäbchenförmigen Kristallits verstanden werden. Dort, wo von einem mittleren Teilchendurchmesser die Rede ist, wird ein volumengemittelter Wert verstanden.

Als wasserlösliche Tenside werden im Rahmen der vorliegenden Erfindung alle oberflächenaktiven Substanzen verstanden, die sich durch einen lipophilen Alkyl-, Alkylphenyl- oder Acylrest mit 8 - 22 C-Atomen und eine hydrophile, ionische oder nichtionische Gruppe auszeichnen, die dem Tensid eine Wasserlöslichkeit von mehr als 1 g/l (20° C) verleiht. Als anionische Tenside sind z.B. die Alkali- oder Ammoniumsalze von C₈-C₁₈-Alkancarbonsäuren (Seifen), von Alkyl-(C₁₂-C₁₈)-Schwefelsäurehalbestern (Alkylsulfate), von Alkylpolyglycolether-schwefelsäurehalbestern (Ethersulfate), von Sulfobernsteinsäuremono-C₈-C₁₈-alkylestern (Sulfosuccinate), von Alkansulfonsäuren (Alkansulfonate), von C₁₂-C₁₈-Acyloxyethansulfonsäuren (Isethionate), von C₁₂-C₁₈-Acylaminoalkansulfonsäuren (Tauride), von N-C₁₂-C₁₈-Acylsarkosin (Sarkosinate), von Alkylpolyglycolethercarbonsäuren (Ethercarboxylate), von Alkyl-(polyglycolether)-phosphorsäureestern (Alkyl-(polyglycolether)-phosphat) geeignet.

Als kationische Tenside sind z.B. Alkyl-trimethylammoniumchlorid, Alkyl-dimethylbenzyl-ammoniumchlorid, Alkylpyridiniumchlorid, Alkyl-dimethyl-hydroxyethylammonium-chlorid, Acylimidazolinium-methosulfate und Acyloxyethyl-trimethylammoniumchlorid geeignet.

Als zwitterionische Tenside eignen sich z.B. Betaintenside wie z.B. Alkyl-dimethylcarboxymethyl-betain und Acylaminoalkyl-dimethyl-carboxymethyl-betain.

Auch Amphotenside wie z.B. Alkylaminopropan-carbonsäure eignen sich als ionische Tenside.

Bevorzugt geeignet sind jedoch die nichtionischen Tenside, insbesondere die Anlagerungsprodukte von Ethylenoxid an Lipide mit beweglichen Wasserstoffatomen. Solche geeigneten nichtionischen Tenside sind z.B. die Anlagerungsprodukte von 6 - 60 Mol Ethylenoxid an lineare Fettalkohole, an Fettsäuren, an Fettamine, an Fettsäuremonoglyceride, an Sorbitan-fettsäuremonoester, an Alkylphenole, an Zucker-Fettsäuremonoester, an Methylglucosid-Fettsäuremonoester sowie an Fettsäuremonoethanolamide. Weitere bevorzugt geeignete nichtionische Tenside sind die Alkyl(oligo)glucoside, die durch Umsetzung von Glucose mit C₈-C₁₈-Fettalkoholen oder durch Umacetalisierung von Butyl-(oligo)-glucosid mit Fettalkoholen zugänglich sind. Bevorzugt geeignete Alkyl-(oligo)-glucoside sind z.B. die Alkyl-(C₈-C₁₆)-glucoside mit mittleren Oligomerisationsgraden (des Glucosidrestes) von 1 bis 2. Solche Produkte sind z.B. unter der Handelsbezeichnung Plantacare®1200 oder Plantacare®600 im Handel. Weitere bevorzugt geeignete nichtionogene Tenside sind die durch Ethoxylierung von gehärtetem Rizinusöl erhältlichen Gemische, die z.B. durch Anlagerung von 30, 40 oder 60 Mol Ethylenoxid an gehärtetes Rizinusöl erhalten werden.

Schließlich sind auch Aminoxid-Tenside und Zucker-Fettsäureester als nichtionogene Tenside geeignet.

Als wasserlösliche polymere Schutzkolloide werden hochmolekulare Verbindungen verstanden, die an die Oberfläche der Nanopartikel adsorbiert werden und diese so modifizieren, daß sie an der Koagulation-und Agglomeration gehindert werden. Geeignete polymere Schutzkolloide sind z.B. natürliche wasserlösliche Polymere wie z.B. Gelatine, Casein, Albumin, Stärke, Pflanzengumme und wasserlösliche Derivate von wasserunlöslichen polymeren Naturstoffen wie z.B. Celluloseether (Methylcellulose, Hydroxyethylcellulose, Carboxymethylcellulose), Hydroxyethyl-Stärke oder Hydroxypropyl-Guar.

Synthetische wasserlösliche Polymere, die sich als Schutzkolloide eignen, sind z.B. Polyvinylalkohol, Polyvinylpyrrolidon, Polyacrylsäuren, Polyasparaginsäure und andere.

Die erfindungsgemäßen Suspensionen werden durch Fällungsreaktionen aus wäßrigen Lösungen wasserlöslicher Calciumsalze und wäßrigen Lösungen wasserlöslicher Phosphat- oder Fluoridsalze hergestellt, dabei wird die Fällung in Gegenwart von wasserlöslichen Tensiden oder wasserlöslichen polymeren Schutzkolloiden durchgeführt. Dies kann z.B. in der Weise erfolgen, daß die Tenside oder Schutzkolloide der wäßrigen Phosphat- oder Fluorid-Salzlösung oder der Lösung des Calciumsalzes vor der Umsetzung beigefügt werden. Alternativ kann die wäßrige Calcium-Salzlösung gleichzeitig mit der Phosphat- oder Fluorid-Salzlösung in eine wäßrige Tensid- oder Schutzkolloid-Lösung eingegeben werden.

Eine weitere Verfahrensvariante besteht darin, daß man die Fällung aus einer stark sauren Lösung eines wasserlöslichen Calciumsalzes und einer stöchiometrischen Menge eines wasserlöslichen Phosphatsalzes mit einem pH-Wert unterhalb von 3 durch Anheben des pH-Werts mit wäßrigem Alkali oder Ammoniak in Gegenwart von wasserlöslichen Tensiden oder wasserlöslichen polymeren Schutzkolloiden durchführt.

Die Konzentration der erfindungsgemäßen Suspensionen an schwerlöslichem Calciumsalz kann einen weiten Bereich von ca. 1 bis 40 Gew.-% umfassen, dabei läßt sich der Gehalt einerseits bei der Herstellung durch die Konzentration der wasserlöslichen Salze, andererseits nach der Fällungsreaktion durch Aufkonzentrieren, z.B. durch Filtration oder Zentrifugation oder durch Abdestillation eines Teils des Wassers erhöhen, ohne daß die Wirkung des Tensids oder des Schutzkolloids dabei verlorengeht.

Die Konzentration des Tensids oder des polymeren Schutzkolloids in der wäßrigen Suspension beträgt z.B. 0,1 bis 20 Gew.-%, bevorzugt 0,1 - 10 Gew.-%, bezogen auf den Gehalt an schwerlöslichem Calciumsalz. In einer bevorzugten Ausführung enthält die erfindungsgemäße Suspension daher 1 - 40 Gew.-% der schwerlöslichen Calciumsalze und zur Stabilisierung 0,1 - 10 Gew.-% eines wasserlöslichen Tensids oder eines wasserlöslichen polymeren Schutzkolloids, bezogen auf das Gewicht des Calciumsalzes.

Bevorzugt geeignet zur Stabilisierung gegen Agglomeration sind vor allem die nichtionischen Tenside in einer Menge von 1 bis 10 Gew.-%, bezogen auf das Gewicht des Calciumsalzes. Als besonders wirksam haben sich die nichtionischen Tenside vom Typ der Alkyl-C₈-C₁₆-(oligo)-glucoside und der Ethoxylate des gehärteten Rizinusöls erwiesen. Diese können zur Stabilisierung auch mit den polymeren Schutzkolloiden zusammen eingesetzt werden.

Zur Herstellung erfindungsgemäßer Suspensionen in anderen flüssigen Medien geht man zweckmäßigerweise von erfindungsgemäßen wäßrigen Suspensionen aus, befreit diese durch Filtration oder Zentrifugation von der wäßrigen Phase, trocknet gegebenenfalls die Nanopartikel und redispergiert sie in organischen Lösungsmitteln. Dabei ist eine erneute Zugabe von Tensiden oder Schutzkolloiden nicht mehr erforderlich, da die Nanopartikel die zur Hemmung der Agglomeration erforderlichen Mengen der Stabilisatoren an der Oberfläche adsorbiert enthalten. Feinteiligkeit und Stabilität solcher Suspensionen ist daher mit der wäßriger Suspensionen vergleichbar. Eine andere Möglichkeit besteht darin, die wäßrige Suspension mit einem höhersiedenden Lösungsmittel, z.B. mit Glycerin, zu mischen und das Wasser destillativ zu entfernen. Als organisches flüssiges Medium eignet sich, insbesondere im Hinblick auf die Verwendung in Zahnpflegemitteln, vor allem Glycerin und dessen flüssige Gemische mit Sorbit und ggf. mit Wasser.

Die erfindungsgemäßen Suspensionen, insbesondere die von Hydroxylapatit, Fluorapatit und Calciumfluorid, eignen sich als remineralisierende Komponente zur Herstellung von Zusammensetzungen zur Reinigung und Pflege der Zähne. Durch die besondere Feinteiligkeit kann die an sich bekannte Wirkung der Festigung des Zahnschmelzes und des Verschlusses von Läsionen und Dentinkanälchen besonders rasch und vollständig erfolgen. Die Zusammensetzungen zur Reinigung und Pflege der Zähne können dabei in Form von Pasten, flüssigen Cremes, Gelen oder Mundspülungen vorliegen. Selbst in flüssigen Zubereitungen verteilen sich die erfindungsgemäßen Suspensionen leicht und die Calciumsalze bleiben stabil dispergiert und neigen nicht zur Sedimentation.

Eine bevorzugte Ausführungsform sind jedoch Zahnpasten mit einem Gehalt an Kieselsäure-Poliermitteln, Feuchthaltemitteln, Bindemitteln und Aromen, die 0,1 - 5 Gew.-% feinteilige Calciumsalze aus der Gruppe Hydroxylapatit, Fluorapatit und Calciumfluorid in Form einer erfindungsgemäßen Suspension enthalten.

Die Zubereitungen zur Reinigung und Pflege der Zähne können dabei die üblichen Komponenten und Hilfsmittel solcher Zusammensetzungen in den dafür üblichen Mengen enthalten. Für Zahnpasten sind dies z.B.
- Putz- und Polierkörper wie z.B. Kreide, Kieselsäuren, Aluminiumhydroxid, Aluminiumsilikate, Calciumpyrophosphat, Dicalciumphosphat, unlösliches Natriummetaphosphat oder Kunstharzpulver
- Feuchthaltemittel wie z.B. Glycerin, 1,2-Propylenglycol, Sorbit, Xylit und Polyethylenglycole
- Bindemittel und Konsistenzregler, z.B. natürliche und synthetische wasserlösliche Polymere und wasserlösliche Derivate von Naturstoffen, z.B. Celluloseether, Schichtsilikate, feinteilige Kieselsäuren (Aerogel-Kieselsäuren, pyrogene Kieselsäuren)
- Aromen, z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen
- Süßstoffe wie z.B. Saccharin-Natrium, Natrium-cyclamat, Aspartame, Acesulfan K, Steviosid, Monellin, Glycyrrhicin, Dulcin, Lactose, Maltose oder Fructose
- Konservierungsmittel und antimikrobielle Stoffe wie z.B. p-Hydroxybenzoesäureester, Natriumsorbat, Triclosan, Hexachlorphen, Phenylsalicylsäureeter, Thymol usw.
- Pigmente wie z.B. Titandioxid oder Pigmentfarbstoffe zur Erzeugung farbiger Streifen
- Puffersubstanzen z.B. primäre, sekundäre oder tertiäre Alkaliphosphate, Citronensäure/Na-Citrat
- wundheilende und entzündungshemmende Wirkstoffe, z.B. Allantoin, Harnstoff, Azulen, Panthenol, Acetylsalicylsäure-Derivate, Pflanzenextrakte, Vitamine, z.B. Retinol oder Tocopherol.

Die folgenden Beispiele sollen den Erfindungsgegenstand näher erläutern:

### Beispiele

### 1. Herstellung von Suspensionen schwerlöslicher Calciumsalze

### 1.1 Herstellung einer Hydroxylapatit-Suspension durch Ausfällung und Redispergierung

50,86 g Ca(NO₃)₂ · 4 H₂O wurden in VE-Wasser gelöst und auf 200 ml aufgefüllt. Dazu wurde 10 g Plantacare 1200® gegeben. Dann wurde 60 ml 25 %ige Ammoniaklösung zugegeben, so daß der pH bei 12 lag.
17 g Ammoniumhydrogenphosphat wurden in VE-Wasser gelöst und auf 200 ml aufgefüllt. Dazu wurde 10 g Plantacare 1200® gegeben. Dann wurde 60 ml 25 %ige Ammoniaklösung zugegeben.
Beide Lösungen wurden auf 75° C gebracht und unter starkem Rühren vermischt. Nach einer Stunde Rühren wurde der Niederschlag abzentrifugiert, mehrmals mit Wasser gewaschen und anschließend in Wasser aufgenommen, so daß eine 5 Gew.-%ige Hydroxylapatitsuspension entstand. Die Teilchengrößen lagen bei 4 - 10 nm x 60 - 130 nm (Durchmesser x Länge).
(VE = vollentsalzt)

### 1.2 Herstellung einer Hydroxylapatit-Suspension durch Umfällung (pH-Verschiebung) und Einengen

25,43 g Ca(NO₃)₂ · 4 H₂O wurden in VE-Wasser gelöst und auf 100 ml aufgefüllt. Ebenso wurden 8,5 g Ammoniumhydrogenphosphat in VE-Wasser gelöst und auf 100 ml aufgefüllt. Die Lösungen wurden unter Bildung eines voluminösen Niederschlags zusammgengegeben. Zu der Suspension wurde 37 %ige Salzsäure zugetropft, bis sich der Niederschlag bei pH 2 vollständig gelöst hatte.
Eine Mischung aus 200 ml VE-Wasser, 200 ml 25 %iger Ammoniaklösung und 20 g Cremophor RH 60® (BASF, Ricinusöl + 60 EO) wurde vorgelegt. Bei 0° C wurde die Apatitlösung in diese Lösung unter Rühren eingetropft, wobei Niederschlagsbildung eintrat. Überschüssiger Ammoniak wurde destillativ abgetrennt, anschließend wurde über Dialyse nitratfrei gewaschen. Durch Einengen am Rotationsverdampfer wurde eine 10 Gew.-%ige Suspension von Hydroxylapatit hergestellt. Die Teilchengrößen lagen bei 30 nm (volumengemittelt) im Durchmesser. (Bestimmung mit dem Micro-Trac 3.150 Ultrafine Particle Analyzer 150 durch Mittelung über das Gesamt-Teilchenvolumen).

### 1.3 Herstellung einer Suspension von Hydroxylapatit analog Beispiel 1.2 (ausgehend von CaCl₂)

11,95 g Calciumchlorid wurden in VE-Wasser gelöst und auf 100 ml aufgefüllt. Ebenso wurden 7,4 g Ammoniumhydrogenphosphat in VE-Wasser gelöst und auf 100 ml aufgefüllt. Die Lösungen wurden unter Bildung eines voluminösen Niederschlags zusammengegeben. Zu der Suspension wurde 37 %ige Salzsäure zugetropft, bis sich der Niederschlag bei pH 2 vollständig gelöst hat.
Eine Mischung aus 200 ml VE-Wasser, 200 ml 25 %iger Ammoniaklösung und 20 g Cremophor RH 60® (BASF, Ricinusöl + 60 EO) wurde vorgelegt. Bei 0° C wurde die Apatitlösung in diese Lösung unter Rühren eingetropft, wobei Niederschlagsbildung eintrat. Überschüssiger Ammoniak wurde detillativ abgetrennt, anschließend wurde über Dialyse nitratfrei gewaschen. Durch Einengen am Rotationsverdampfer wurde eine 10 Gew.-%ige Suspension von Hydroxylapatit hergestellt. Die Teilchengrößen lagen bei 10 - 35 nm x 20 - 50 nm (Durchmesser x Länge).

### 1.4 Herstellung einer Hydroxylapatit-Suspension analog Beispiel 1.2 unter Verwendung von Arlatone 289 (BASF)

Anstelle von 20 g Cremophor RH 60 wurden 35 g Arlatone 289 eingesetzt. Es wurde eine 10 Gew.-%ige Suspension von Hydroxylapatit mit einer mittleren Partikelgröße von 40 nm erhalten. (Micro-Trac 3.150 Ultrafine Particle Analyzer).

### 1.5 Herstellung einer Hydroxylapatit-Suspension in Glycerin

0,3 Mol Calciumchlorid wurden in 2000 ml VE-Wasser gelöst und auf 25° C temperiert. Mit Ammoniaklösung wurde ein pH von 12 eingestellt. Unter starkem Rühren wurde anschließend eine auf 25° C temperierte und mit Ammoniak auf pH 10 eingestellte Lösung von 0,18 Mol Ammoiniumhydrogenphosphat in 400 ml VE-Wasser langsam zugetropft. Nach 20 h Reaktionszeit wurde 3 g Cremophor RH 60®-Lösung (40 Gew.-%ig in VE-Wasser) zugegeben und durch Eintrag mechanischer Energie (Rühren, Ultraschall) dispergiert. Anschließend wurde die Suspension mehrmals abzentrifugiert und zunächst mit 1 %iger wäßriger Cremophor RH 60®-Lösung, dann mit Ethanol gewaschen. Anschließend wurde das Material in 100 ml Glycerin aufgenommen. In dieser glycerinischen Suspension lagen Hydroxylapatit-Partikel mit Größen von 5 - 20 nm x 10 - 70 nm (Durchmesser x Länge) vor.

### 1.6 Herstellung einer Suspension von fluordotiertem Hydroxylapatit in Glycerin

0,3 Mol Calciumchlorid wurden in 2000 ml VE-Wasser gelöst und auf 25° C temperiert. Mit Ammoniaklösung wurde ein pH von 12 eingestellt. Hierzu wurde eine Lösung von 2,27 g Ammoniumfluorid in 50 ml VE-Wasser gegeben. Unter starkem Rühren wurde anschließend eine auf 25° C temperierte und mit Ammoniak auf pH 10 eingestellte Lösung von 0,18 Mol Ammoniumhydrogenphosphat in 400 ml VE-Wasser langsam zugetropft. Nach 20 h Reaktionszeit wurde 3 g Cremophor RH 60®-Lösung (40 Gew.-%ig in VE-Wasser) zugegeben und durch Eintrag mechanischer Energie (Rühren, Ultraschall) dispergiert. Anschließend wurde die Suspension mehrmals abzentrifugiert und zunächst mit 1 %iger wäßriger Cremophor RH 60®-Lösung, dann mit Ethanol gewaschen. Anschließend wurde das Material in 100 ml Glycerin aufgenommen. Dabei wurde eine glycerinische Suspension von Ca₅(PO₄)₃(OH,F)-Partikeln mit einer Größe von 5 - 20 nm x 10 - 70 nm (Durchmesser x Länge) erhalten.

### 1.7 Herstellung einer Calciumfluorid-Suspension durch Ausfällung

11,95 g wasserfreies CaCl₂ wurde in VE-Wasser gelöst und auf 100 ml aufgefüllt. In einer Vorlage wurden 200 ml VE-Wasser, 35g Arlatone 289 (BASF) sowie 15 g Ammoniumfluorid vermischt. Beide Lösungen wurden auf 0° C abgekühlt und die erste Lösung unter starkem Rühren in die zweite gegeben. Die gebildete Dispersion wurde am Rotationsverdampfer bei 70° C eingeengt bis der Feststoffgehalt bei 10 Gew.-% lag. Anschließend wurde mittels Dialyse gewaschen. Dabei wurde eine Calciumfluorid-Suspension mit einer mittleren (volumengewichtet) Teilchengröße von 20 nm erhalten.

### 2. Zahncremes mit Calciumsalz-Nanopartikeln

| **Rezeptur-Beispiele** | **2.1** | **2.2** |
|---|---|---|
| Sident® 8 | 10,0 Gew.-% | 10,0 Gew.-% |
| Sident® 22S | 7,0 Gew.-% | 7,0 Gew.-% |
| Sipernat® 320DS | 0,8 Gew.-% | 0,8 Gew.-% |
| CaF₂-Suspension Beispiel 1.7 | 5,0 Gew.-% | - |
| Hydroxylapatit-Suspension Beispiel 1.1 | - | 5,0 Gew.-% |
| Polywachs 1550 | 2,0 Gew.-% | 2,0 Gew.-% |
| Texapon K 1296 | 1,5 Gew.-% | 1,5 Gew.-% |
| Titandioxid | 1,0 Gew.-% | 1,0 Gew.-% |
| Cekol 500 T | 1,0 Gew.-% | 1,0 Gew.-% |
| Na-Fluorid | 0,33 Gew.-% | 0,33 Gew.-% |
| Na-Benzoat | 0,25 Gew.-% | 0,25 Gew.-% |
| Aroma | 1,0 Gew.-% | 1,0 Gew.-% |
| Tagat S | 0,2 Gew.-% | - |
| Na-Saccharinat | 0,15 Gew.-% | 0,15 Gew.-% |
| Tri-Natriumphosphat | 0,10 Gew.-% | 0,10 Gew.-% |
| Sorbit (70 %ig in Wasser) | 31,0 Gew.-% | 31,0 Gew.-% |
| Wasser | ad 100 Gew.-% | ad 100 Gew.-% |

Es wurden folgende Handelsprodukte verwendet:
- Plantacare®1200 :: C₁₂-C₁₆-Fettalkohol-oligo-(1.4)-glucosid
ca. 50 Gew.-% in Wasser
Hersteller: HENKEL KGaA
- Cremophor®RH 60 :: Rizinusöl(hydriert)-poly(60)-glycolether
Hersteller: BASF
- Arlatone®289 :: Rizinusöl(hydriert)-poly(54)-glycolether
Hersteller: Atlas Chemie (ICI)
- Sident®8 :: Synth. amorph. Kieselsäure, BET 60 m²/g
Stampfdichte: 350 g/l Hersteller: DEGUSSA
- Sident®22 S :: Hydrogelkieselsäure, BET 140 m²/g
Stampfdichte: 100 g/l
Hersteller: DEGUSSA
- Polywachs® 1550 :: Polyethylenglycol, MG: 1550
Erweichungspunkt 45 - 50° C
Hersteller: RWE / DEA
- Texapon®K 1296 :: Natrium-Laurylsulfat-Pulver
Hersteller: HENKEL KGaA
- Cekol®500 T :: Natrium-Carboxymethylcellulose
Viskosität (2 %ig in Wasser, Brookfield LVF
20° C) : 350 - 700 mPa·s
Lieferant: Nordmann-Rassmann
- Tagat® S :: Polyoxyethylen-(20)-glycerylmonostearat
Hersteller: Tego Cosmetics (Goldschmidt)

## Patentansprüche

1. Suspensionen von wenig wasserlöslichen Calciumsalzen, ausgewählt aus Phosphaten, Fluoriden und Fluorophosphaten in einem flüssigen Medium, in dem diese Salze nicht oder wenig löslich sind, **dadurch gekennzeichnet, daß** die Calciumsalze in Form von Primärteilchen mit Durchmessern von 5 bis 50 Nanometern und Längen von 10 bis 150 Nanometern vorliegen und durch einen Gehalt von wenigstens 0,01 Gew.-%, bezogen auf das Gewicht der Suspension, eines wasserlöslichen Tensids oder eines wasserlöslichen polymeren Schutzkolloids gegen Agglomeration stabilisiert sind.

2. Suspensionen gemäß Patentanspruch 1, **dadurch gekennzeichnet, daß** 1 bis 40 Gew.-% der schwerlöslichen Calciumsalze und zur Stabilisierung 0,1 bis 10 Gew.-%, bezogen auf das Gewicht des schwerlöslichen Calciumsalzes, eines wasserlöslichen Tensids oder eines wasserlöslichen polymeren Schutzkolloids in der Suspension enthalten sind.

3. Suspensionen nach einem der Ansprüche 1 oder 2, **dadurch gekennzeichnet, daß** zur Stabilisierung nichtionische Tenside in einer Menge von 1 bis 10 Gew.-%, bezogen auf das Gewicht des schwerlöslichen Calciumsalzes, enthalten sind.

4. Verfahren zur Herstellung der Suspensionen gemäß Anspruch 1 - 3, durch Fällungsverfahren aus wäßrigen Lösungen wasserlöslicher Calciumsalze und wäßrigen Lösungen wasserlöslicher Phosphat- oder Fluoridsalze, **dadurch gekennzeichnet, daß** die Fällung in Gegenwart von wasserlöslichen Tensiden oder wasserlöslichen polymeren Schutzkolloiden durchgeführt wird.

5. Verfahren zur Herstellung der Suspensionen gemäß Anspruch 1 - 3 durch Fällung aus einer sauren Lösung eines wasserlöslichen Calciumsalzes und einer stöchiometrischen Menge eines wasserlöslichen Phosphatsalzes mit einem pH-Wert unterhalb 3 durch Anheben des pH-Wertes mit wäßrigen Alkalien oder Ammoniak in Gegenwart von wasserlöslichen Tensiden oder wasserlöslichen polymeren Schutzkolloiden.

6. Verwendung der Suspensionen gemäß einem der Ansprüche 1 - 3 zur Herstellung von Zusammensetzungen zur Reinigung und Pflege der Zähne wobei die Suspensionen als remineralisierende Komponente verwendet werden.

7. Zahnpasten mit einem Gehalt an Kieselsäure-Poliermitteln, Feuchthaltemitteln, Bindemitteln und Aromen, **dadurch gekennzeichnet, daß** 0,1 - 5 Gew.-% feinteiliger Calciumsalze aus der Gruppe amorphes Calciumphosphat, Hydroxylapatit, Fluorapatit und Calciumfluorid in Form einer Suspension gemäß Anspruch 1 - 3 enthalten sind.

## Claims

1. Suspensions of poorly water-soluble calcium salts selected from phosphates, fluorides and fluorophosphates in a liquid medium in which these salts are insoluble or poorly soluble, **characterized in that** the calcium salts are present in the form of primary, particles with diameters of 5 to 50 nanometres and lengths of 10 to 150 nanometres and are stabilized against agglomeration by a content of at least 0.01% by weight, based on the weight of the suspension, of a water-soluble surfactant or a water-soluble polymeric protective colloid.

2. Suspensions as claimed in claim 1, **characterized in that** they contain 1 to 40% by weight of the poorly soluble calcium ,salts and, for stabilization, 0.1 to 10% by weight, based on the weight of the poorly soluble calcium salt, of a water-soluble surfactant or a water-soluble polymeric protective colloid.

3. Suspensions as claimed in claim 1 or 2, **characterized in that** they contain 1 to 10% by weight, based on the weight of the poorly soluble calcium salt, of nonionic surfactants for stabilization.

4. A process for the production of the suspensions claimed in claims 1 to 3 by precipitation from aqueous solutions of water-soluble calcium salts and aqueous solutions of water-soluble phosphate or fluoride salts, **characterized in that** precipitation is carried out in the presence of water-soluble surfactants or water-soluble polymeric protective colloids.

5. A process for the production of the suspensions claimed in claims 1 to 3 by precipitation from an acidic solution of a water-soluble calcium salt and a stoichiometric quantity of a water-soluble phosphate salt with a pH below 3 by raising the pH with aqueous alkalis or ammonia in the presence of water-soluble surfactants or water-soluble polymeric protective colloids.

6. The use of the suspensions claimed in any of claims 1 to 3 for the production of tooth-cleaning and dental care compositions, the suspensions being used as a remineralizing component.

7. Toothpastes containing silica polishing components, humectants, binders and flavours, **characterized in that** 0.1 to 5% by weight of fine-particle calcium salts from the group of amorphous calcium phosphate, hydroxylapatite, fluoroapatite and calcium fluoride are present in the form of the suspension claimed in any of claims 1 to 3.

## Revendications

1. Suspensions de sels de calcium peu hydrosolubles, choisis parmi les phosphates, les fluorures et les fluorophosphates dans un milieu liquide, dans lequel ces sels ne sont pas solubles ou sont peu solubles, **caractérisées en ce que** les sels de calcium se présentent sous la forme de particules primaires ayant des diamètres de 5 à 50 nanomètres et des longueurs de 10 à 150 nanomètres, et sont stabilisées envers l'agglomération par une teneur d'au moins 0,01 % en poids, par rapport au poids de la suspension, d'un tensio-actif hydrosoluble ou d'un colloïde protecteur polymère hydrosoluble.

2. Suspensions selon la revendication 1, **caractérisées en ce que** 1 à 40% en poids des sels de calcium peu solubles et 0,1 à 10% en poids, par rapport au poids du sel de calcium peu soluble, d'un tensio-actif hydrosoluble ou d'un colloïde protecteur polymère hydrosoluble, pour la stabilisation, sont contenus dans la suspension.

3. Suspensions selon l'une quelconque des revendications 1 ou 2, **caractérisées en ce que**, pour la stabilisation, des tensio-actifs non ioniques y sont contenus en une quantité de 1 à 10% en poids, par rapport au poids du sel de calcium peu soluble.

4. Procédé de préparation des suspensions selon la revendication 1 à 3, à l'aide d'un procédé de précipitation dans des solutions aqueuses de sels de calcium hydrosolubles et des solutions aqueuses de sels phosphate ou fluorure hydrosolubles, **caractérisé en ce que** la précipitation est réalisée en présence de tensio-actifs hydrosolubles ou de colloïdes protecteurs polymères hydrosolubles.

5. Procédé de préparation des suspensions selon la revendication 1 à 3, par précipitation dans une solution acide d'un sel de calcium hydrosoluble et d'une quantité stochiométrique d'un sel phosphate hydrosoluble ayant une valeur de pH inférieure à 3, par élévation de la valeur de pH avec des alcalis aqueux ou de l'ammoniaque en présence de tensio-actifs hydrosolubles ou de colloïdes protecteurs polymères hydrosolubles.

6. Utilisation des suspensions selon l'une quelconque des revendications 1 à 3, pour préparer des compositions destinées au nettoyage et au soin des dents, les suspensions étant employées comme composant re-minéralisant.

7. Pâtes dentifrices ayant une teneur en agents de polissage à base d'acide silicique, en agents humidifiants, en liants et en substances aromatiques, **caractérisées en ce que** 0,1 à 5% en poids de sels de calcium finement divisés issus du groupe formé par le phosphate de calcium amorphe, l'hydroxyapatite, la fluoroapatite, et le fluorure de calcium, y sont contenus sous forme d'une suspension selon la revendication 1 à 3.
